# EUROPEAN PATENT SPECIFICATION

(11) **EP 3 876 716 B1**
(45) Date of publication and mention of the grant of the patent: **03.06.2026**
(21) Application number: 19880992.3
(22) Date of filing: 03.10.2019
(51) Int. Cl.: A01N 1/147

(54) **CRYO-CARRIER**
KRYOTRÄGER
SUPPORT CRYOGÉNIQUE

(30) Priority: 09.11.2018 CN 201821847886 U
(43) Date of publication of application: 15.09.2021
(73) Proprietor: Fertility Preservation Canada Ltd., Ottawa, Ontario K1E 0A8 (CA)
(72) Inventor: SONGGUO, Xue, Shanghai 20012 (CN); QIUPING, Peng, Hamilton, Ontario L8S 3W6 (CA); SEANG, Lin Tan, Westmount, Québec H3Y 1P3 (CA)
(74) Representative: Espatent Oy
(86) International application number: PCT/CA2019/000138
(87) International publication number: WO 2020/093133

(56) References cited:
- JP-A- 2015 002 681
- NAKASHIMA, A ET AL.: "Optimization of a Novel Nylon Mesh Container for Human Embryo Ultrarapid Vitrification", FERTIL. STERIL., vol. 93, no. 7, 1 May 2010 (2010-05-01), pages 2405 - 2410, XP027021102, ISSN: 1556-5653
- MATSUMOTO, H ET AL.: "Vitrification of Large Quantities of Immature Bovine Oocytes Using Nylon Mesh", CRYOBIOLOGY, vol. 42, no. 2, March 2001 (2001-03-01), pages 139 - 144, XP008141836, ISSN: 0011-2240, DOI: 10.1006/CRYO.2001.2309
- KIM, SH ET AL.: "Simplified EM Grid Vitrification is a Convenient and Efficient Method for Mouse Mature oocyte Cryopreservation", YONSEI MED J, vol. 47, no. 3, 30 June 2006 (2006-06-30), pages 399 - 404, XP055707643, ISSN: 0513-5796
- CHU, D ET AL.: "Effects of Different Open Cryo-carriers on Embryo Survival and Clinical Outcomes in Frozen Embryo Transfer Cycle Pateints", SYST BIOL REPROD MED, vol. 64, no. 2, April 2008 (2008-04-01), pages 138 - 145, XP055707645, ISSN: 1939-6376, DOI: 10.1080/19396368.2017.1419510
- SARGUSTY, J ET AL.: "Current Progress in Oocyte and Embryo Cryopreservation by Slow Freezing and Vitrification", REPRODUCTION, vol. 141, no. 1, January 2011 (2011-01-01), pages 1 - 19, XP055068580, ISSN: 1741-7899, DOI: 10.1530/REP-10-0236
- MANDAWALA, AA ET AL.: "Cryopreservation of Animal Oocytes and Embryos: Current Progress and Future Prospects", THERIOGENOLOGY, vol. 86, no. 7, 15 October 2016 (2016-10-15), pages 1637 - 1644, XP029725202, ISSN: 1879-3231, DOI: 10.1016/j.theriogenology.2016.07.018

## Description

### TECHNICAL FIELD

The present disclosure relates to the field of assisted reproduction, more particularly to a cryo-carrier.

### BACKGROUND

In the field of assisted reproduction, human or animal embryos, zygotes, eggs, tissues (e.g., ovaries) and the like need to be frozen for long-term storage (hereinafter collectively referred to as embryos for convenience of description). The emerging ultra-rapid freezing method is the vitrification freezing method, which freezes and thaw embryos at a rate of about 20000°C per minute, resulting in good embryo survival and developmental potential. During the freezing process the embryo needs to be loaded on a specific cryo-carrier for freezing. The types of cryo-carrier currently widely used clinically include, but are not limited to, Cryotop^{™}, Cryolock^{™}, Cryotech^{™}, Strawtop^{™}, Cryoleaf^{™}, Rapid-i^{™}, Cryloop^{™}, Cryotip^{™} and the like. Among them, Cryotop^{™}, Cryolock^{™}, Cryotech^{™}, Strawtop^{™} and Cryoleaf^{™} are classified as solid surface methods. Rapid-i^{™} and Cryoloop^{™} are a ring or hole and are classified as a ring method in which embryos are frozen depending on liquid surface tension attached to the ring or hole.

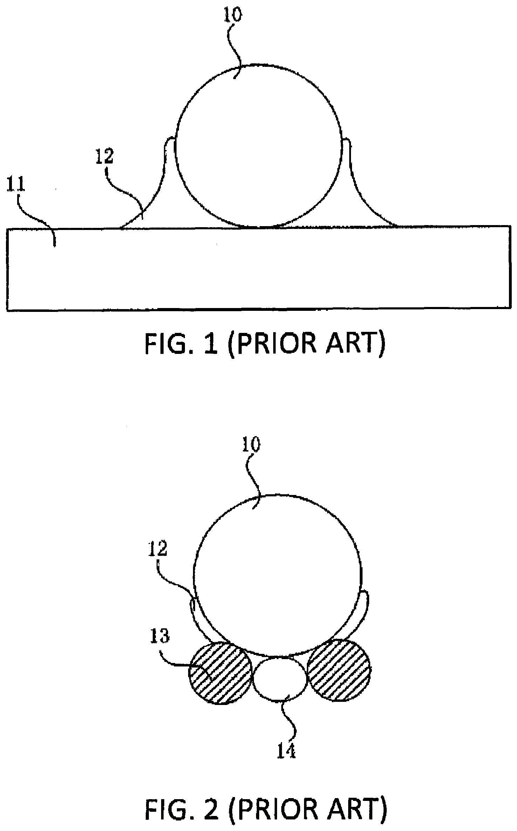

Solid surface methods are most widely used, and embryos are frozen on an elongated plastic sheet. The plastic sheet has a thickness of about 100 µm, a width of about 2 to 5 mm, and a length of 1 to 2 cm. The rear end of the plastic sheet is connected to a long plastic rod for hand-held operation. Referring to FIG.1, there is shown a schematic view of an embryo placed on a prior art plastic sheet. When the embryo 10 is placed on the plastic sheet 11, the contact surface between the embryo 10 and the plastic sheet 11 is considered to be insulated thus reducing the cooling rate, but the cooling rate can be increased with less residual cooling solution 12 around the embryo 10. In the ring method, the embryo can conduct heat in four directions to increase the cooling rate, but the cooling rate is reduced by more residual cooling solution around the embryo.

Some scholars tried to replace plastic sheets with nylon mesh. FIG.2 shows a schematic view of an embryo placed on a prior art nylon mesh. In this method, the embryo 10 is placed on the nylon mesh 13, and less cooling solution 12 remains around the embryo to increase the cooling rate. However, this method has the following disadvantages: (1) the nylon mesh 13 is soft and inconvenient to operate, (2) the nylon filament refraction during thawing greatly affects the positioning of embryos and (3) the nylon mesh 13 is thicker (the thickness of the mesh is the diameter of the nylon wire, and is about 50 µm), and air bubbles 14 can be formed in the nylon wire mesh when the nylon mesh is frozen and thawed, which is equivalent to forming a heat insulation layer below the embryo 10, so that the cooling rate is reduced. Therefore, the method taking the nylon mesh as the carrying rod has little clinical application.

Accordingly, there is a clear need for a cryo-carrier that can ensure the cooling rate and is convenient to operate.

JP 2015-2681 A discloses a cryo-carrier for freezing at least one embryo, wherein a vitrification liquid absorber such as a paper or a nonwoven fabric is arranged on a metal support body. In the embryo placement portion a hole may be provided in the metal support body in order to allow for observation using a transmission microscope.

### SUMMARY

The invention is defined in the appended claims.

There is provided a cryo-carrier, which comprises a carrier region formed by a plurality of grids, wherein the carrier region comprises an embryo placing portion and a non-embryo placing portion, and the non-embryo placing portion surrounds the embryo placing portion. In the non-embryo placing portion, each grid is filled with a supporting sheet so that the hardness of the non-embryo placing portion is stronger than that of the embryo placing portion, thereby supporting the embryo placing portion. This structure ensures a uniform cooling rate.

In one embodiment, a nylon mesh forms the carrier region.

In one embodiment, in the embryo placement portion, the thickness of the grid is less than one tenth of the diameter of the embryo to be placed thereupon.

In one embodiment, the thickness of the mesh in the embryo placement portion ranges from 1 to 40 µm.

In one embodiment, in the embryo placement portion, the side length of the grid Is less than one third of the diameter of the embryo.

In one embodiment, in the embryo placement portion, the grid has a side length in the range of 36 to 50 µm.

In one embodiment, the area of the embryo placement area is (300-1000 µm) * (300-1000 µm).

In one embodiment, in the non-embryonic placement region, the thickness of the support sheet is greater than or equal to the thickness of the mesh.

In one embodiment, the support sheet has a thickness of 10 to 100 µm.

In one embodiment, the cryo-carrier further includes a grip rod connected to one end of the carrier region.

An advantage of the disclosed cryo-carrier is that the hardness of the non-embryo placing portion is much stronger than that of the embryo placing portion, so that the non-embryo placing portion can support the embryo placing portion, and the cooling rate is ensured and the manutention of the cryo-carrier Is convenient.

Another advantage of the disclosed cryo-carrier is that because the embryo is placed In a smaller embryo placement portion, the magnification of the micro-mirror can be increased during thawing to accurately position the embryo.

A further advantage of the disclosed cryo-carrier is that the lower part of the embryo can be probed under the grid, the lower part of the embryo is directly in contact with the liquid nitrogen during freezing, no bubble heat insulation layer is formed, the grid is four-way heat transfer, the cooling rate is increased. In addition, the cryo-carrier allows easy removal and replacement of the cooling solution, thus making It compatible with automatic embryo freezing and thawing technologies, which require replacement of the cooling solution during the freezing and thawing process.

### BRIEF DESCRIPTION OF THE FIGURES

Embodiments of the disclosure will be described by way of examples only with reference to the accompanying drawing, in which:
**FIG.1** is a schematic cross-sectional diagram of an embryo placed on a prior art plastic sheet;
**FIG.2** is a schematic cross-sectional diagram of an embryo placed on a prior art nylon mesh;
**FIG.3** is a schematic structural diagram of a cryo-carrier according to an illustrative embodiment of the present disclosure;
**FIG.4** is a schematic structural diagram of the carrier region of the cryo-carrier of FIG. 3; and
**FIG.5** is a schematic cross-sectional diagram of an embryo placed on the embryo placement portion of the carrier region of FIG. 4.

Similar references used in different Figures denote similar components.

### DETAILED DESCRIPTION

Generally stated, the non-limitative illustrative embodiments of the present disclosure provide a cryo-carrier, which comprises a carrier region formed by a plurality of grids, wherein the carrier region comprises an embryo placing portion and a non-embryo placing portion, and the non-embryo placing portion surrounds the embryo placing portion. In the non-embryo placing portion, each grid Is filled with a supporting sheet so that the hardness of the non-embryo placing portion is stronger than that of the embryo placing portion, thereby supporting the embryo placing portion. This structure ensures a uniform cooling rate.

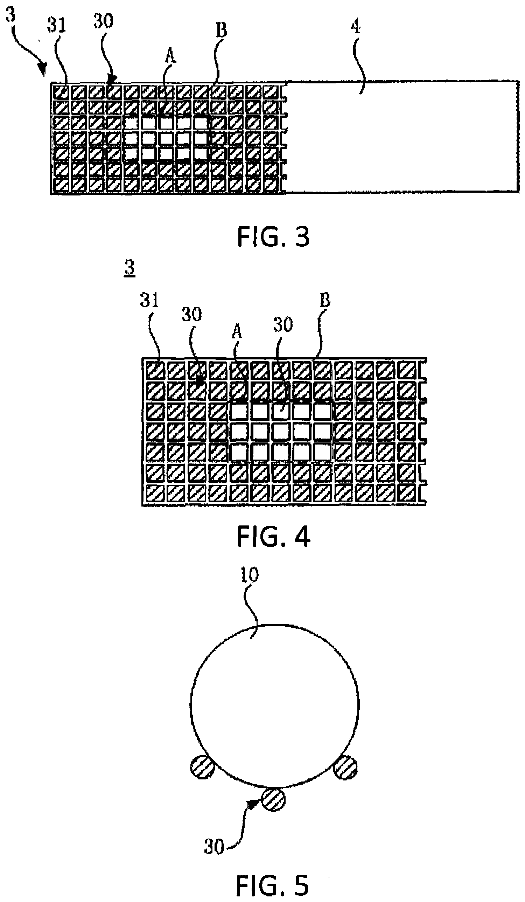

Referring to FIGS. 3 and 4, there is shown the cryo-carrier according to an illustrative embodiment of the present disclosure, the cryo-carrier comprises a carrier region 3 consisting of a plurality of grids 30. For example, in the present Illustrative embodiment, the carrier region 3 is formed by a nylon mesh. The nylon mesh refers to a net structure formed by a plurality of nylon wires in a staggered way.

The carrier region 3 includes an embryo placement portion A for placing one or more embryo and a non-embryo placement portion B, wherein the non-embryo placement portion B surrounds the embryo placement portion A *in* order to provide support. The embryo placement portion A is schematically encircled by a dashed box FIGS. 3 and 4, the number of grids in the embryo placement portion A not representing its actual number.

In the non-embryo placement portion B, each of the grids 30 is filled with a support sheet 31 so that the hardness of the non-embryo placement portion B is greater than that of the embryo placement portion A, thereby supporting the embryo placement portion A. In FIGS. 3 and 4, the support sheet 31 is schematically depicted by hatched lines. In particular, the support sheet 31 may be formed In the grid 30 of the non-embryo placement portion B using methods such as 3D printing or mold pouring, and the materials of the support sheet 31 include, but are not limited to, thermoplastic materials such as plastics, nylon, and the like. In the non-embryo placement portion B, due to the existence of the support sheet 31, a plurality of grids 30 are firmly connected with each other, which results in the hardness of the non-embryo placement portion B being higher than that of the embryo placement portion A.

In the illustrative embodiment the thickness of the support sheet 31 is greater than or equal to the thickness of the grid 30 to further increase the hardness of the non-embryo placement portion B. The thickness of the supporting sheet 31 in the illustrative embodiment may vary from 10 to 100 µm, including all values and sub ranges in between. However, in alternative embodiments the thickness of the supporting sheet 31 may vary in accordance with the thickness and/or wire inter-spacing of grids designed for specific applications (for example, for supporting various types of animal embryos, which differ in size compared to human embryos).

Referring to FIG.5, there is shown a cross-sectional diagram of an embryo 10 placed on the embryo placement portion A. The cryo-carrier having a grid structure, the contact area of the embryo 10 and the grid 30 is small, the adhesion force is small, and holes are arranged below the embryo 10, so that the embryo 10 can be in contact with thawing solution, therefore, the embryo 10 is easy to float in the thawing solution, and the thawing solution can be moved to make the thawing solution in the hole below the embryo 10 flush the embryo 10, so that the embryo 10 can be easily separated from the cryo-carrier, and the thawing solution can be easily replaced and removed, so that the cryo-carrier of the can be very conveniently applied to the automatic technology of embryo freezing and thawing.

In the illustrative embodiment, the area of the embryo placement portion A is from 300 to 1000 µm by 300 to 1000 µm. This represents an advantage over prior art embryo carrier rods that have an embryo placement area of about 2000 µm by 5000 µm, which require decreasing the magnification of the microscope or moving the carrier rod in order to bring the embryos into the visual field of the microscope. The area of the embryo placement portion A of the cryo-carrier, and thus the visual field, are greatly reduced, so that the magnification of the microscope can be increased to clearly see the embryo, thereby realizing easy positioning.

In the illustrative embodiment, the wire Inter-spacing of the grid 30 Is in the range of 36 µm to 50 µm, and preferably less than one third of the diameter of the embryo to be placed on the embryo placement portion A. Furthermore, in the illustrative embodiment the thickness of the grid 30 is in the range of 1 µm to 40 µm, and preferably less than one tenth of the diameter of the embryo to be placed on the embryo placement portion A. It is to be understood that in alternative embodiments the wire inter-spacing and the thickness of the grid 30 may vary depending on the application.

Referring still to FIG.5, since the thickness of the grid 30 is small and the wire inter-spacing of the grid 30 is less than the diameter of the embryo 10, the lower portion of the embryo 10 may be probed below the mesh after the embryo 10 is placed in the embryo placement portion A, no bubbles are generated between the bottom of the embryo 10 and the grid 30, and the lower part of the embryo 10 is directly in contact with liquid nitrogen during the freezing operation, i.e. no insulating layer is formed, thereby increasing the cooling rate. In addition, since the embryo 10 is disposed on the grid 30, the grid 30 can transfer heat in four directions, further increasing the cooling rate.

Referring back to FIG.3, in the illustrative embodiment the cryo-carrier further includes a grip rod 4 connected to the carrier region 3. When the cryo-carrier is in use, such as when it is displaced, a user can hold the grip rod 4 to operate the cryo-carrier. The cryo-carrier also includes other structures conventionally used in the art, such as a sleeve for protecting the carrier region 3, and the like (not shown).

### EXAMPLE APPLICATIONS

Application 1: Manual artificial freezing and thawing of embryos.
   1. Artificial freezing of embryos: The embryos are placed in the embryo placement area and excess cooling solution VS is aspirated from below with a tissue, the cryo-carrier is put into liquid nitrogen and cryopreserved.
   2. Artificial thawing of embryos: The cryo-carrier is removed from liquid nitrogen and inserted into a thawing solution. The embryo is automatically detached from the cryo-carrier, or the thawing solution is reciprocally moved left and right to detach the embryo from the cryo-carrier.
Application 2: Freeze and thaw tissue.
   1. Freeze tissue: The tissue is placed in the embryo placement area and excess cooling solution VS is aspirated from below with a tissue, the cryo-carrier Is put into liquid nitrogen and cryopreserved.
   2. Thawing tissue: The cryo-carrier is removed from liquid nitrogen and thawed by insertion Into a thawing solution.
Application 3: Embryo freezing automation.
   1. The embryo is placed in the embryo placement portion A, the cryo-carrier is turned 180 degrees and cooling solution ES is added unto the embryo placement region A from above.
   2. Wait for 5-15 minutes and add cooling solution VS unto the embryo placement region A from above.
   3. Wait for about 30 seconds and remove the cooling solution from above with a tissue.
   4. Add cooling solution VS from above.
   5. The cryo-carrier is turned 180 degrees and adding cooling solution VS is added from above.
   6. Wait for about 1 minute and the cooling solution is aspirated from below with a tissue.
   7. The cryo-carrier is put into liquid nitrogen and cryopreserved.

It is to be understood that the cryo-carrier may also be used to freeze other biological matter, such as, for example, tissues,

Although the present disclosure has been described with a certain degree of particularity and by way of an illustrative embodiment and examples thereof, it is to be understood that the present disclosure is not limited to the features of the embodiments described and illustrated herein, but includes all variations and modifications within the scope of the invention as defined by the claims.

## Claims

1. A cryo-carrier for freezing at least one embryo, comprising:
a carrier region (3) formed by a plurality of grids (30), the carrier region (3) having an embryo placement portion (A) for supporting the at least one embryo, the grids in the embryo placement portion (A) forming holes allowing a lower part of the embryo to be probed and to be in contact with a thawing solution, and a non-embryo placement portion (B) surrounding the embryo placement portion (A);
each grid in the non-embryo placement portion (B) being filled with a support sheet (31) so that the non-embryo placement portion (B) is stronger than the embryo placement portion (A), thereby supporting the embryo placement portion (A).

2. A cryo-carrier according to claim 1, wherein the at least one embryo has a diameter and wherein the plurality of grids (30) have a thickness that is less than one tenth of the diameter of the at least one embryo.

3. A cryo-carrier according to either of claims 1 or 2, wherein the carrier region (3) is made of a nylon mesh.

4. A cryo-carrier according to claim 3, wherein the thickness of the nylon mesh in the embryo placement portion (A) ranges from 1 to 40 µm.

5. A cryo-carrier according to any one of claims 1 to 4, wherein the at least one embryo has a diameter and wherein the plurality of grids (30) have a wire inter-spacing that is less than one third of the diameter of the at least one embryo.

6. A cryo-carrier according to claim 5, wherein the wire inter-spacing of the plurality of grids (30) in the embryo placement portion (A) ranges from 36 to 50 µm.

7. A cryo-carrier according to any of claims 1 to 6, wherein the area of the embryo placement portion (A) ranges from 300 to 1000 µm by 300 to 1000 µm.

8. A cryo-carrier according to any of claims 1 to 7, wherein the thickness of the support sheet (31) is greater than or equal to the thickness of the plurality of grids (30).

9. A cryo-carrier according to any of claims 1 to 7, wherein the support sheet (31) has a thickness that ranges from 10 to 100 µm.

10. A cryo-carrier according to any of claims 1 to 9, wherein the cryo-carrier further comprises a grip rod (4) connected at an end of the carrier region (3).

11. A cryo-carrier according to any of claims 1 to 10, wherein the support sheet (31) is formed in the plurality of grids (30) of the non-embryo placement portion (B) using 3D printing or mold pouring, and wherein the materials of the support sheet (31) include thermoplastic materials.

## Patentansprüche

1. Kryoträger zum Einfrieren mindestens eines Embryos, umfassend:
einen Trägerbereich (3), der aus einer Mehrzahl von Gittern (30) besteht, wobei der Trägerbereich (3) einen Embryo-Ablageabschnitt (A) zur Auflagerung des mindestens einen Embryos, wobei die Gitter im Embryo-Ablageabschnitt (A) Löcher bilden, die es ermöglichen, dass ein unterer Teil des Embryos sondiert wird und mit einer Auftaulösung in Kontakt ist, und einen embryofreien Ablageabschnitt (B), der den Embryo-Ablageabschnitt (A) umgibt, aufweist;
wobei jedes Gitter im embryofreien Ablageabschnitt (B) mit einer flächigen Stützeinlage (31) gefüllt ist, so dass der embryofreie Ablageabschnitt (B) fester als der Embryo-Ablageabschnitt (A) ist und dadurch den Embryo-Ablageabschnitt (A) abstützt.

2. Kryoträger nach Anspruch 1, wobei der mindestens eine Embryo einen Durchmesser aufweist und wobei die mehreren Gitter (30) eine Dicke aufweisen, die kleiner als ein Zehntel des Durchmessers des mindestens einen Embryos ist.

3. Kryoträger nach einem der Ansprüche 1 oder 2, wobei der Trägerbereich (3) aus einem Nylon-Gitterstoff gefertigt ist.

4. Kryoträger nach Anspruch 3, wobei die Dicke des Nylon-Gitterstoffs im Embryo-Ablageabschnitt (A) im Bereich von 1 bis 40 µm liegt.

5. Kryoträger nach einem der Ansprüche 1 bis 4, wobei der mindestens eine Embryo einen Durchmesser aufweist und wobei die mehreren Gitter (30) einen Drahtzwischenabstand aufweisen, der kleiner als ein Drittel des Durchmessers des mindestens einen Embryos ist.

6. Kryoträger nach Anspruch 5, wobei der Drahtzwischenabstand der mehreren Gitter (30) im Embryo-Ablageabschnitt (A) im Bereich von 36 bis 50 µm liegt.

7. Kryoträger nach einem der Ansprüche 1 bis 6, wobei die Fläche des Embryo-Ablageabschnitts (A) im Bereich von 300 bis 1000 µm mal 300 bis 1000 µm liegt.

8. Kryoträger nach einem der Ansprüche 1 bis 7, wobei die Dicke der flächigen Stützeinlage (31) größer als oder gleich der Dicke der mehreren Gitter (30) ist.

9. Kryoträger nach einem der Ansprüche 1 bis 7, wobei die flächige Stützeinlage (31) eine Dicke im Bereich von 10 bis 100 µm aufweist.

10. Kryoträger nach einem der Ansprüche 1 bis 9, wobei der Kryoträger ferner eine an einem Ende des Trägerbereichs (3) angebrachten Griffstange (4) umfasst.

11. Kryoträger nach einem der Ansprüche 1 bis 10, wobei die flächige Stützeinlage (31) in den mehreren Gittern (30) des embryofreien Ablageabschnitt (B) mittels 3D-Druck oder Formgießen ausgebildet wird, und wobei die Materialien der flächigen Stützeinlage (31) thermoplastische Werkstoffe umfassen.

## Revendications

1. Cryoporteur pour la congélation d'au moins un embryon, comprenant :
une zone porteuse (3) formée par une pluralité de grilles (30), la zone porteuse (3) ayant une partie de placement d'embryon (A) pour soutenir ledit au moins un embryon, les grilles dans la partie de placement d'embryon (A) formant des trous permettant à une partie inférieure de l'embryon d'être sondée et d'être en contact avec une solution de décongélation, et une partie de placement non embryonnaire (B) entourant la partie de placement d'embryon (A);
chaque grille dans la partie de placement non embryonnaire (B) étant remplie d'une feuille de support (31) de sorte que la partie de placement non embryonnaire (B) soit plus résistante que la partie de placement d'embryon (A), soutenant ainsi la partie de placement d'embryon (A).

2. Cryoporteur selon la revendication 1, dans lequel ledit au moins un embryon a un diamètre et dans lequel la pluralité de grilles (30) a une épaisseur qui est inférieure à un dixième du diamètre dudit au moins un embryon.

3. Cryoporteur selon l'une des revendications 1 ou 2, dans lequel la zone porteuse (3) est faite d'un tissu de nylon à mailles.

4. Cryoporteur selon la revendication 3, dans lequel l'épaisseur du tissu de nylon à mailles dans la partie de placement d'embryon (A) est comprise entre 1 et 40 µm.

5. Cryoporteur selon l'une des revendications 1 à 4, dans lequel ledit au moins un embryon a un diamètre et dans lequel la pluralité de grilles (30) a un espacement entre les fils qui est inférieur à un tiers du diamètre dudit au moins un embryon.

6. Cryoporteur selon la revendication 5, dans lequel l'espacement entre les fils de la pluralité de grilles (30) dans la partie de placement d'embryon (A) est compris entre 36 et 50 µm.

7. Cryoporteur selon l'une des revendications 1 à 6, dans lequel l'aire de la partie de placement d'embryon (A) est comprise dans la plage de 300 à 1000 µm fois 300 à 1000 µm.

8. Cryoporteur selon l'une des revendications 1 à 7, dans lequel l'épaisseur de la feuille de support (31) est supérieure ou égale à l'épaisseur de la pluralité de grilles (30).

9. Cryoporteur selon l'une des revendications 1 à 7, dans lequel la feuille de support (31) a une épaisseur comprise entre 10 et 100 µm.

10. Cryoporteur selon l'une des revendications 1 à 9, dans lequel le cryoporteur comprend également une tige de préhension (4) reliée à une extrémité de la zone porteuse (3).

11. Cryoporteur selon l'une des revendications 1 à 10, dans lequel la feuille de support (31) est formée dans la pluralité de grilles (30) de la partie de placement non embryonnaire (B) au moyen d'une impression 3D ou d'un moulage, et dans lequel les matériaux de la feuille de support (31) comprennent des matériaux thermoplastiques.
